(19) Europäisches Patentamt
European Patent Office
Office européen des brevets

(11) Publication number: 0 168 826 B1

(12) EUROPEAN PATENT SPECIFICATION

(45) Date of publication of patent specification: 04.05.88

(51) Int. Cl.[4]: C 07 C 47/22, C 07 C 45/38, C 07 C 57/05

(21) Application number: 85108950.8

(22) Date of filing: 17.07.85

# (54) Process for producing methacrolein and methacrylic acid.

(30) Priority: 18.07.84 JP 148768/84
20.07.84 JP 150897/84
23.07.84 JP 152599/84
24.07.84 JP 153377/84

(43) Date of publication of application: 22.01.86 Bulletin 86/04

(45) Publication of the grant of the patent: 04.05.88 Bulletin 88/18

(84) Designated Contracting States: BE DE FR GB NL

(56) References cited:
EP-A-0 058 046
EP-A-0 068 193
DD-A- 207 706
DE-A-2 441 109
DE-A-2 550 962
DE-A-2 941 341

The file contains technical information submitted after the application was filed and not included in this specification

(73) Proprietor: MITSUBISHI RAYON CO. LTD.
3-19, Kyobashi 2-chome Chuo-Ku
Tokyo 104 (JP)

(72) Inventor: Oh-kita, Motomu
c/o Mitsubishi Rayon Co., Ltd. 20-1 Miyuki-cho
Otake-shi Hiroshima (JP)
Inventor: Kita, Teruo
Mitsubishi Rayon Co., Ltd. 3-19, Kyobashi 2-chome
Chuo-ku Tokyo (JP)

(74) Representative: Patentanwälte TER MEER - MÜLLER - STEINMEISTER
Mauerkircherstrasse 45
D-8000 München 80 (DE)

(56) References cited:
DE-A-3 001 911
US-A-4 421 938

PATENT ABSTRACTS OF JAPAN, unexamined applications, C field, vol. 1, no. 48, May 11, 1977 THE PATENT OFFICE JAPANESE GOVERNMENT page 158 C 77

EP 0 168 826 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the invention

The present invention relates to a process for synthesizing methacrolein and methacrylic acid by catalytically oxidizing isobutyl alcohol in the gas phase with molecular oxygen in the presence of a transition metal catalyst.

Description of the background

Various processes have been proposed for synthesizing methacrolein and methacrylic acid by the catalytic oxidation or catalytic oxidative dehydrogenation of isobutylene, tert-butyl alcohol, isobutylaldehyde and/or isobutyric acid in the gas phase.

From DD—A—207 706 a process for the continuous manufacture of methacrolein and methacrylic acid is known wherein organic tert.-butyl compounds and precursor of isobutene, such as isobutanol are oxidized in the gas phase with molecular oxygen in the presence of a transition metal catalyst necessarily comprising bismuth as one of the component elements. The oxidation of isobutyl alcohol provides for a conversion of the alcohol of 98.1% and a selectivity to methacrolein and methacrylic acid respectively of 33.0% and only 0.7%.

Summary of the invention

Accordingly, it is the object of the present invention to provide a method for synthesizing methacrolein and methacrylic acid by the gas phase oxidation of isobutyl alcohol wherein a complete alcohol conversion is attained and the selectivity to methacrylic acid enhanced.

This object has been solved by the characterizing features of the process according to claim 1.

The invention provides a process for synthesizing methacrolein and methacrylic acid by catalytically oxidizing isobutyl alcohol in the gas phase with molecular oxygen in the presence of a transition metal catalyst wherein the catalyst used has the empirical formula:

$$P_aMo_bX_cZ_dO_e$$

wherein X is at least one member selected from the group consisting of at least one alkali metal, such as sodium, potassium, rubidium or cesium, or thallium, Z is at least one member selected from the group consisting of zinc, aluminum, antinomy, sulfur, indium, uranium, cadmium, calcium, silver, chromium, silicon, germanium, cobalt, zirconium, tin, strontium, selenium, tunsten, tantalum, titanium, iron, tellurium, copper, thorium, lead, niobium, nickel, vanadium, barium, arsenic, beryllium, boron, magnesium, manganese, samarium, lanthanum, rhodium, ruthenium, rhenium, platinum and palladium, a=0.01—8, b=12, c=0.01—5, d=0—20 and the value of e is determined by the oxidation states of the metal atoms in the catalyst.

The catalyst may be prepared by any known process such as the evaporation-to-dryness process or the precipitation process.

In the preparation of the catalyst, a carrier such as silica, alumina or silica/alumina, may be used to support the active materials.

In the catalytic oxidation which occurs in the gas phase, the starting isobutyl alcohol is preferably diluted with an inert gas.

The oxygen source which effects the oxidation of isobutyl alcohol may be pure oxygen or it may be air. Air is preferred from the industrial viewpoint. The reaction pressure ranges from atmospheric pressure to several atmospheres. The reaction temperature is variable in the range of 150 to 450°C, and either a fluidized bed or fixed bed system may be employed.

Having generally described this invention, a further understanding can be obtained by reference to certain specific examples which are provided herein for purposes of illustration only and are not intended to be limiting unless otherwise specified.

In the following examples, the conversion of isobutyl alcohol and the selectivities for methacrolein and methacrylic acid are defined as follows:

$$\text{Conversion of isobutyl alcohol (\%)} = \frac{\text{number of moles of reacted isobutyl alcohol}}{\text{number of moles of fed isobutyl alcohol}} \times 100$$

$$\text{Methacrolein selectivity (\%)} = \frac{\text{number of moles of formed methacrolein}}{\text{number of moles of reacted isobutyl alcohol}} \times 100$$

$$\text{Methacrylic acid selectivity (\%)} = \frac{\text{number of moles of formed methacrylic acid}}{\text{number of moles of reacted isobutyl alcohol}} \times 100$$

In the following examples, parts are given by weight and the analysis were performed by gas chromatography.

Example 1

200 parts of ammonium molybdate was added to 1,000 parts of water, and the material was heated with stirring. 10.9 parts of 85% phosphoric acid and then 9.5 parts of potassium nitrate were added thereto, and the mixture was evaporated to dryness by heating with stirring. The product was further dried at 130°C for 16 h.

The resulting solid was finely pulverized, compression-molded and calcined at 380°C for 4 h and a catalyst product was obtained. The catalyst had a composition of $P_1Mo_{12}K_1$.

The catalyst was placed in a reactor of a fixed bed type and a gaseous mixture of 5% of isobutyl alcohol, 10% of oxygen, 20% of steam and 65% of nitrogen was introduced therein at a space velocity of 2400 $h^{-1}$, while the temperature was kept at 340°C to conduct the reaction.

The conversion of isobutyl alcohol was 100% and the selectivities for methacrolein and methacrylic acid were 33.8% and 7.3%, respectively.

Example 2

200 parts of ammonium molybdate and 12 parts of ammonium metavanadate were added to 1,000 parts of water and the material was heated with stirring. 11.9 parts of 85% phosphoric acid and then 10.4 parts of potassium nitrate were added thereto and the resulting mixture was evaporated to dryness by heating with stirring. The product was further dried at 130°C for 16 h.

The resulting solid was finely pulverized, compression-molded and calcined at 380°C for 4 h to obtain a catalyst. The catalyst had a composition of $P_1Mo_{11}V_1K_1$.

The reaction was carried out under the same reaction conditions as in Example 1 to obtain an isobutyl alcohol conversion of 100% and selectivities for methacrolein and methacrylic acid of 34.1% and 7.5%, respectively.

Example 3

200 parts of ammonium molybdate and 8.8 parts of ammonium metavanadate were added to 1,000 parts of water and the material was heated with stirring. Then, 16.3 parts of 85% phosphoric acid and 1.8 parts of boric acid were added thereto. A solution of 9.1 parts of copper nitrate in 30 parts of water and then 5.5 parts of cesium nitrate were added thereto. The resulting mixture was evaporated to dryness by heating with stirring. The product was further dried at 130°C for 16 h. The resulting solid was finely pulverized, compression-molded and calcined at 380°C for 4 h to obtain a catalyst. The catalyst had a composition of

$$P_{1.5}Mo_{12}V_{0.8}Cu_{0.4}B_{0.3}Cs_{0.3}.$$

The reaction was carried out under the same reaction conditions as in Example 1 to obtain an isobutyl alcohol conversion of 100% and selectivities for methacrolein and methacrylic acid of 35.3% and 8.6%, respectively.

Examples 4 to 24

Isobutyl alcohol was oxidized in the same manner as described in Example 1 in the presence of each of the catalysts shown in Table 1 which had been prepared in the same manner as described in Example 3, except that the catalyst components were varied. The results are shown in Table 1.

TABLE 1

| Example | Catalyst | Isobutyl alcohol conversion (%) | Selectivity (%) | |
|---|---|---|---|---|
| | | | Methacrolein | Methacrylic acid |
| 4 | $P_1Mo_{10}V_1W_1Cr_{0.1}Cu_{0.4}Te_{0.5}K_{0.5}$ | 100 | 34.5 | 8.9 |
| 5 | $P_2Mo_{12}Mg_{0.2}Zn_1Tl_{0.8}$ | 100 | 32.8 | 10.0 |
| 6 | $P_2Mo_{12}Zr_1Sm_{0.3}Re_{0.05}Rb_1$ | 100 | 34.3 | 9.8 |
| 7 | $P_2Mo_{12}Sb_1Ta_1Ag_{0.1}K_1$ | 100 | 32.9 | 7.0 |
| 8 | $P_{1.5}Mo_{12}V_{0.8}Al_1S_{0.5}K_1$ | 100 | 33.0 | 7.0 |
| 9 | $P_{1.5}Mo_{12}V_{0.8}Nb_{0.5}In_{0.3}K_1$ | 100 | 32.7 | 6.8 |
| 10 | $P_2Mo_{12}As_1Cr_{0.1}Cu_{0.4}K_{0.5}$ | 100 | 34.1 | 8.5 |
| 11 | $P_2Mo_{12}V_{0.8}Cu_{0.4}U_{0.3}K_{0.5}$ | 100 | 34.0 | 7.5 |
| 12 | $P_2Mo_{12}V_{0.8}Cu_{0.4}Cd_{0.3}Si_1K_1$ | 100 | 34.3 | 7.3 |
| 13 | $P_2Mo_{12}V_{0.8}Sn_1Cu_{0.5}Ca_{0.5}K_{0.5}$ | 100 | 33.9 | 6.7 |
| 14 | $P_1Mo_{12}Sr_{0.3}Ti_1Ge_{0.4}K_{0.8}$ | 100 | 34.8 | 7.9 |
| 15 | $P_2Mo_{12}V_{0.8}Fe_{0.5}Mg_{0.5}Ce_{0.3}K_1$ | 100 | 32.9 | 6.1 |
| 16 | $P_1Mo_{12}V_1W_1Cu_{0.4}Th_{0.3}K_1$ | 100 | 32.8 | 6.5 |
| 17 | $P_1Mo_{12}V_1Ta_1Cu_{0.4}Pb_{0.3}K_1$ | 100 | 32.9 | 6.3 |
| 18 | $P_2Mo_{12}V_{0.8}Cu_{0.4}Pd_{0.01}Na_1$ | 100 | 33.0 | 7.8 |
| 19 | $P_1Mo_{12}V_{0.8}Cu_{0.4}Pt_{0.01}K_{0.5}$ | 100 | 34.7 | 7.1 |
| 20 | $P_2Mo_{12}V_{0.8}Mn_{0.5}Sb_{0.5}Rb_{0.8}$ | 100 | 35.0 | 7.2 |
| 21 | $P_2Mo_{12}W_{0.5}Cu_{0.5}Ru_{0.01}Rb_1$ | 100 | 34.5 | 6.9 |
| 22 | $P_2Mo_{12}V_{0.8}Cu_{0.4}Sb_{0.3}Rh_{0.05}K_{0.5}$ | 100 | 35.1 | 7.5 |
| 23 | $P_2Mo_{12}V_1W_1Ba_{0.3}La_{0.3}Cs_{0.5}$ | 100 | 34.3 | 7.2 |
| 24 | $P_{1.5}Mo_{12}V_{0.8}Cu_{0.4}Ag_{0.1}Sb_{0.3}Cs_{0.3}$ | 100 | 35.4 | 7.7 |

Example 25

The catalyst prepared in Example 24 was placed in a reactor of fixed bed type. A gaseous mixture of 4% of isobutyl alcohol, 14% of oxygen, 25% of steam and 57% of nitrogen was introduced therein at a space velocity of 2200 $h^{-1}$, while the temperature was kept at 340°C to conduct the reaction. The isobutyl alcohol conversion was 100% and the selectivities for methacrolein and methacrylic acid were 18.6% and 32.1% respectively.

Example 26

The catalyst prepared in Example 24 was placed in a reactor of the fixed bed type. A gaseous mixture of 4% of isobutyl alcohol, 14% of oxygen, 25% of steam and 57% of nitrogen was introduced therein at a space velocity of 1100 $h^{-1}$, while the temperature was kept at 340°C to conduct the reaction. The isobutyl alcohol conversion was 100% and the selectivities for methacrolein and methacrylic acid were 3.5% and 31.0%, respectively.

**Claims**

1. A process for synthesizing methacrolein and methacrylic acid by catalytically oxidizing isobutyl alcohol in the gas phase with molecular oxygen in the presence of a transition metal catalyst characterized in that the catalyst used has the empirical formula:

$$P_aMo_bX_cZ_dO_e$$

wherein X is at least one member selected from the group consisting of at least one alkali metal or thallium, Z is at least one member selected from the group consisting of zinc, aluminum, antimony, sulfur, indium, uranium, cadmium, calcium, silver, chromium, silicon, germanium, cobalt, zirconium, tin, strontium, selenium, tungsten, tantalum, titanium, iron, tellurium, copper, thorium, lead, niobium, nickel, vanadium, barium, arsenic, beryllium, boron, magnesium, manganese, samarium, lanthanum, rhodium, ruthenium, rhenium, platinum and palladium, a=0.01—8, b=12, c=0.01—5, d=0—20 and the value of e is determined by the oxidation states of the metal atoms in the catalyst.

2. The process of claim 1, characterized in that the temperature of the oxidation reaction is 150° to 450°C.

3. The process of claim 1, characterized in that the source of molecular oxygen is air.

**Patentansprüche**

1. Verfahren zur Synthese von Methacrolein und Methacrylsäure durch katalytische Oxidation von Isobutylalkohol in der Gasphase mit molekularem Sauerstoff in Gegenwart eines Übergangsmetallkatalysators, dadurch gekennzeichnet, daß der eingesetzte Katalysator die empirische Formel

$$P_aMo_bX_cZ_dO_e$$

besitzt, worin X mindestens ein Vertreter der aus mindestens einem Alkalimetall oder Thallium bestehenden Gruppe, Z mindestens ein Vertreter der aus Zink, Aluminium, Antimon, Schwefel, Indium, Uran, Cadmium, Calcium, Silber, Chrom, Silicium, Germanium, Kobalt, Zirkonium, Zinn, Strontium, Selen, Wolfram, Tantal, Titan, Eisen, Tellur, Kupfer, Thorium, Blei, Niob, Nickel, Vanadium, Barium, Arsen, Beryllium, Bor, Magnesium, Mangan, Samarium, Lanthan, Rhodium, Ruthenium, Rhenium, Platin und Palladium bestehenden Gruppe, a=0,01—8, b=12, c=0,01—5, d=0—20 bedeuten und der Wert für e durch die Oxidationsstufen der Metallatome im Katalysator bestimmt wird.

2. Verfahren nach Anspruch 1, dadurch gekennzeichnet, daß die Temperatur der Oxidationsreaktion 150° bis 450°C beträgt.

3. Verfahren nach Anspruch 1, dadurch gekennzeichnet, daß die Quelle für molekularen Sauerstoff Luft ist.

**Revendications**

1. Un procédé la synthèse de la méthacroléine et de l'acide méthacrylique par oxydation catalytique de l'alcool isobutylique en phase gazeuse au moyen d'oxygène moléculaire en présence d'un catalyseur de métal de transition, caractérisé en ce que le catalyseur utilisé présente la formule empirique:

$$P_aMo_bX_cZ_dO_e$$

dans laquelle X est au moins un élément choisi dans le groupe comprenant au moins un métal alcalin ou le thallium, Z est au moins un élément choisi dans le groupe comprenant les suivants: zinc, aluminium, antimoine, soufre, indium, uranium, cadmium, calcium, argent, chrome, silicium, germanium, cobalt, zirconium, étain, strontium, sélénium, tungstène, tantale, titane, fer, tellure, cuivre, thorium, plomb, niobium, nickel, vanadium, baryum, arsenic, béryllium, bore, magnésium, manganèse, samarium, lanthane, rhodium, ruthénium, rhénium, platine et palladium, a=0,01—8; b=12; a=0,01—5; d=0—20; et la valeur de e est déterminée par les états d'oxydation des atomes de métaux dans le catalyseur.

2. Le procédé selon la revendication 1, caractérisé en ce que la température de la réaction d'oxydation est de 150°C à 450°C.

3. Le procédé selon la revendication 1, caractérisé en ce que la source d'oxygène moléculaire est l'air.